# EUROPEAN PATENT APPLICATION

(11) **EP 4 566 538 A1**
(43) Date of publication of application: **11.06.2025**
(21) Application number: 23850001.1
(22) Date of filing: 27.07.2023
(51) Int. Cl.: A61B 5/291, A61B 5/263

(54) **BIOELECTRODE**

(30) Priority: 04.08.2022 JP 2022124857
(71) Applicant: NOK Corporation, Minato-ku Tokyo 105-8585 (JP)
(72) Inventor: KUBO, Masayuki, Fujisawa-shi, Kanagawa 251-0042 (JP); HAYASHI, Taisei, Fujisawa-shi, Kanagawa 251-0042 (JP); HAYASHI, Takahiro, Fujisawa-shi, Kanagawa 251-0042 (JP)
(74) Representative: TBK
(86) International application number: PCT/JP2023/027555
(87) International publication number: WO 2024/029446

(57) **Abstract**

A bioelectrode includes an electrode member and a plurality of cores. The electrode member is made of elastic material with conductivity and includes a base, which has a shape of a sheet or a shape of a plate and includes a first surface and a second surface facing in a direction opposite to a direction in which the first surface faces, and a plurality of protrusions protruding from the second surface. The plurality of cores is made of a material having a Young's modulus greater than that of the elastic material and is provided corresponding to two or more protrusions among the plurality of protrusions. A distal end of each of the plurality of cores is disposed within a middle of a corresponding protrusion in a direction of protrusion.

## Description

### TECHNICAL FIELD

This disclosure relates to bioelectrodes.

### BACKGROUND ART

A bioelectrode, which is to be attached to a surface of a living body, is generally used to acquire biometric information such as an electrocardiogram, an electroencephalogram, or an electromyogram, which is based on a change in electric potential in the living body. For example, a bioelectrode described in Patent Document 1 includes a plate-shaped portion and a plurality of protrusions protruding from the plate-shaped portion. These are made of insulating elastic members. Here, a distal end of each of the plurality of protrusions is covered by a conductive resin layer. In addition, within each of the plurality of protrusions, a conductive wire is disposed that is electrically connected to the conductive resin layer. This conductive wire is made of conductive fibers.

### Related Art Document

### Patent Document

Patent Document 1: WO 2020/095589

### SUMMARY OF THE INVENTION

### Problem to be Solved by the Invention

However, in the bioelectrode described in Patent Document 1, the protrusions are repeatedly used and gradually suffer from fatigue; accordingly, degrees of rigidity of the protrusions are reduced or the protrusions plastically deform. Thus, in a case in which there is hair on a surface of a living body, it is difficult for the protrusions to push aside the hair. As a result, it is not possible to realize a good state of contact between the protrusions and the living body; thus, it is difficult to measure biometric information.

Here, as a countermeasure for reducing fatigue of the protrusions, it is conceivable for the Young's modulus of a material of the protrusions to be increased. However, by this countermeasure, it is not possible to use the flexibility of the protrusions; thus, a problem occurs in that a subject feels discomfort when biometric information is measured.

### Means for Solving Problem

In order to solve the above problem, a bioelectrode according to one aspect of this disclosure includes an electrode member, which is made of an elastic material with conductivity and includes a base having a shape of a sheet or a shape of a plate and including a first surface and a second surface facing in a direction opposite to a direction in which the first surface faces and a plurality of protrusions protruding from the second surface, and a plurality of cores, which is made of a material having a Young's modulus greater than that of the elastic material and being provided corresponding to two or more protrusions among the plurality of protrusions, wherein a distal end of each of the plurality of cores is disposed within a middle of a corresponding protrusion in a direction of protrusion.

### Effect of Invention

According to this disclosure, it is possible to execute measurement of biometric information over a long period in time and to reduce discomfort experienced by a subject when biometric information is measured.

### BRIEF DESCRIPTION OF THE DRAWINGS

Fig. 1 is an exploded perspective view of a bioelectrode according to a First Embodiment.
Fig. 2 is a side view of the bioelectrode according to the First Embodiment.
Fig. 3 is a bottom view of the bioelectrode according to the First Embodiment.
Fig. 4 is a cross section taken along line A-A of Fig. 3.
Fig. 5 is an exploded perspective view of a bioelectrode according to a Second Embodiment.
Fig. 6 is a cross section of the bioelectrode according to the Second Embodiment.
Fig. 7 is an exploded perspective view of a bioelectrode according to a Third Embodiment.
Fig. 8 is a cross section of the bioelectrode according to the Third Embodiment.
Fig. 9 is a diagram showing an example of use of the bioelectrode.

### MODES FOR CARRYING OUT THE INVENTION

In the following, preferred embodiments according to this disclosure will be described with reference to the accompanying drawings. It should be noted that dimensions and scales of elements shown in the drawings may differ from those of actual elements, and some elements may be shown schematically to facilitate understanding. In addition, the scope of this disclosure is not limited to these embodiments unless the following explanation includes a description that specifically limits this disclosure.

### 1. First Embodiment

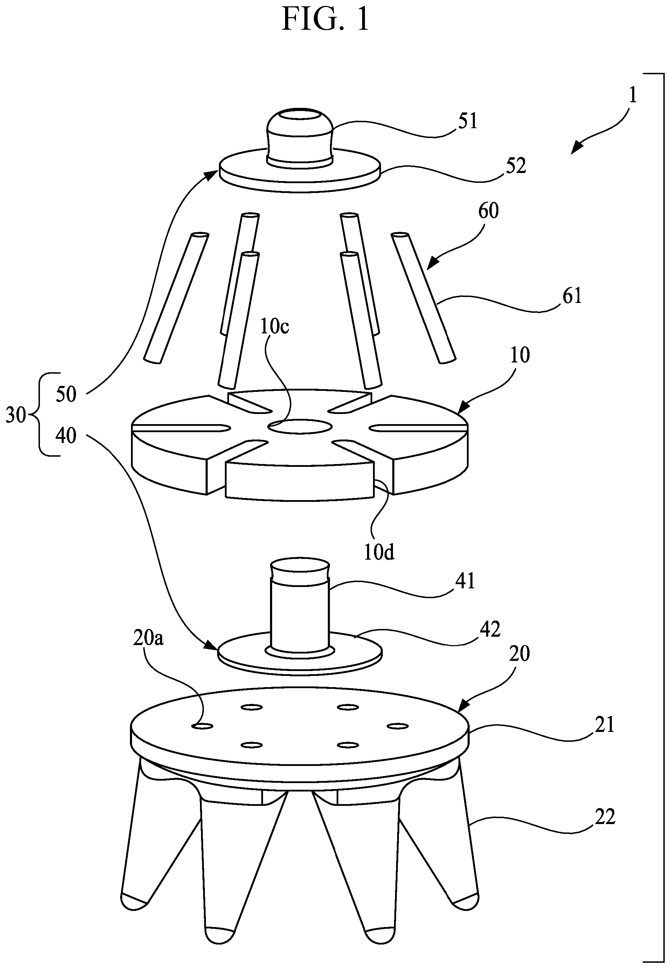

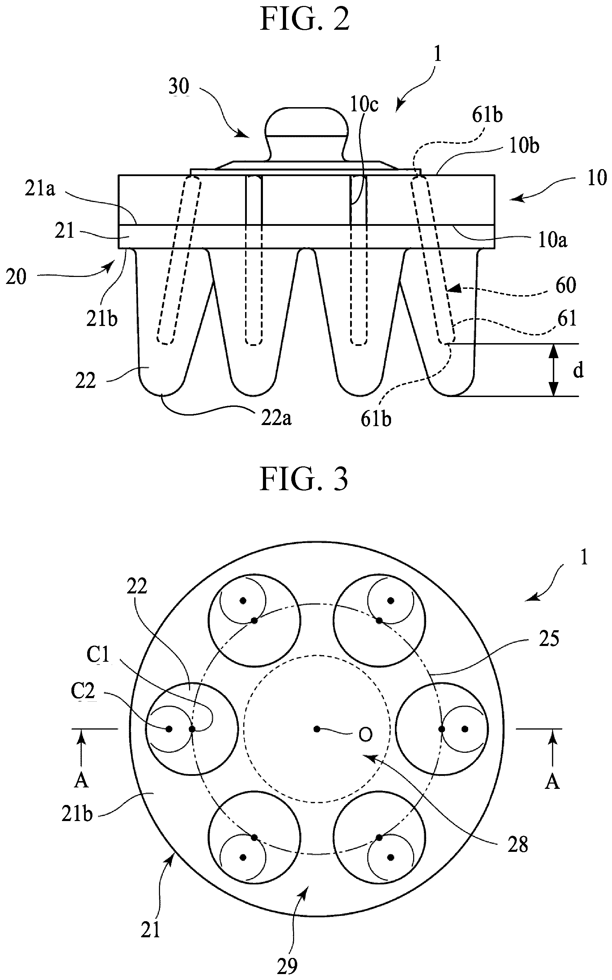

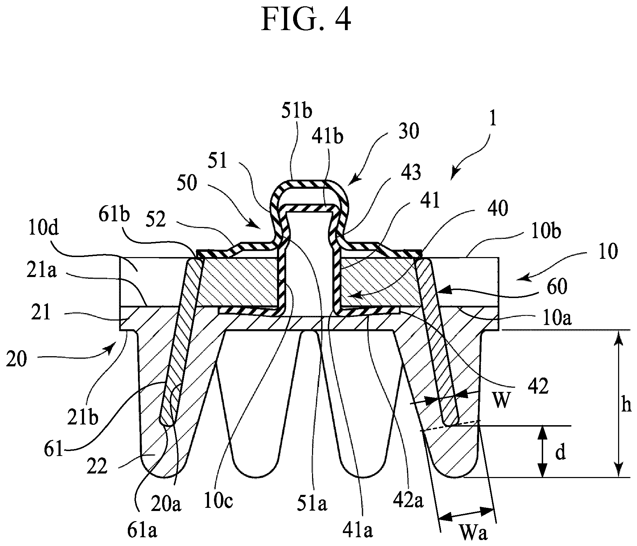

Fig. 1 is an exploded perspective view of a bioelectrode 1 according to a First Embodiment. Fig. 2 is a side view of the bioelectrode 1 according to the First Embodiment. Fig. 3 is a bottom view of the bioelectrode 1 according to the First Embodiment. Fig. 4 is a cross section taken along line A-A of Fig. 3.

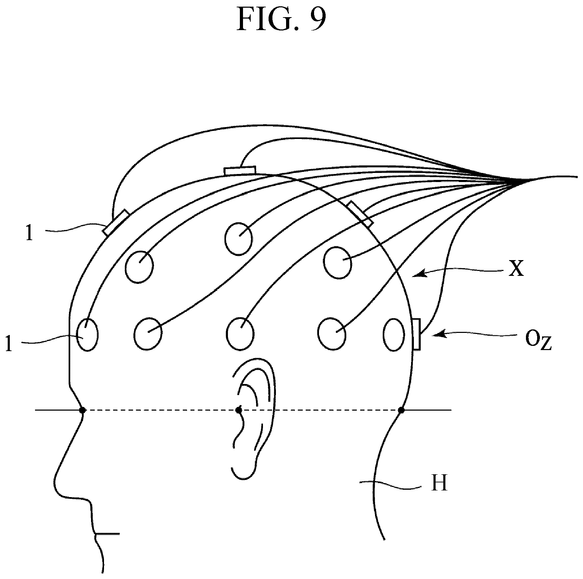

The bioelectrode 1 is an electrode for detecting, as a biometric signal, a change in electric potential in a living body such as a human being or an animal. The bioelectrode 1 is used in a state in which the bioelectrode 1 is in contact with a surface of a living body to be measured. Although described in detail below with reference to Fig. 9, the bioelectrode 1 is suitably used to take an electroencephalogram in a state in which the bioelectrode 1 is in contact with a scalp, for example. Although not shown, the bioelectrode 1 is electrically connected to a measuring device for generating biometric information, such as an electroencephalogram, based on the biometric signal from the bioelectrode 1. In the following, the living body to be measured may be referred to as a subject.

It should be noted that use of the bioelectrode 1 is not limited to taking an electroencephalogram, and it may be measurement of biometric information such as an electrocardiogram or an electromyogram, for example. Thus, a portion of the living body in contact with the bioelectrode 1 is not limited to the scalp, and it may be, for example, an arm, a leg, a chest, or a back or the like, or alternatively, it may not be a region having hair. In addition, use of the bioelectrode 1 is not limited to use for measuring a biometric signal, and it may be a use for providing electrical stimulation to a living body, for example.

As shown in Fig. 1, the bioelectrode 1 includes a support member 10, an electrode member 20, a connector 30, and a core group 60. In the following, each of the elements of the bioelectrode 1 will be described sequentially.

The support member 10 is a member for supporting the electrode member 20. The support member 10 is made of an insulating material, in other words, a dielectric material. Examples of the insulating material include a polymer such as silicone rubber, urethane rubber, fluorine rubber, and terpolymer rubber (EPDM) made from ethylene, propylene, and diene. From a viewpoint of enhancing adhesion between the support member 10 and the electrode member 20, the insulating material is preferably a polymer of the same type as that of a polymer constituting the electrode member 20.

In an example shown in Fig. 1, the support member 10 is discoid. Here, the support member 10 includes a pair of plate surfaces 10a and 10b, a through hole 10c, and a plurality of notches 10d. The plate surface 10a is a surface for supporting the electrode member 20. The plate surface 10b is a surface facing in a direction opposite to a direction in which the plate surface 10a faces. The through hole 10c is a hole passing through the support member 10 in a thickness direction. The through hole 10c is open to each of the plate surface 10a and the plate surface 10b. Each of the plurality of notches 10d is a groove provided to an outer circumferential surface of the support member 10. A direction of depth of each of the plurality of notches 10d is a direction of radius of the support member 10, and each of the plurality of notches 10d extends from one of the plate surface 10a and the plate surface 10b, to the other. In the plurality of notches 10d, a part of the core group 60 is disposed.

It should be noted that, in plan view, a shape of the support member 10 is not limited to the example shown in Fig. 1, and it may be a polygon, for example. Each of the plate surface 10a and the plate surface 10b is not limited to a flat surface, and it may be a convex curved surface, a concave curved surface, or a conical surface, for example. A transverse-sectional shape of the through hole 10c is not limited to a circle, and it may be a polygon such as a square or a pentagon, for example. The support member 10 may be provided, or may be omitted, as appropriate, or it may be integrated with the electrode member 20.

The electrode member 20 is a conductive member to be in contact with the surface of the living body. The electrode member 20 is made of an elastic material with conductivity. The elastic material includes a rubber material and a conductive particle, for example. Examples of the rubber material include silicone rubber, terpolymer rubber (EPDM) made from ethylene, propylene, and diene, nitrile rubber, and urethane rubber and the like. Among them, silicone rubber is suitably used as the rubber material, from a viewpoint of biocompatibility, etc. Examples of the conductive particle include a carbon particle, which is made of carbon black, carbon nanotubes, or graphite or the like, a metallic particle, which is made of metal such as silver, and a particle, which is made of a metal compound such as silver chloride. It should be noted that the elastic material may include not only the rubber material and the conductive particle but also a fiber-based material, such as a nonwoven fabric made of a resin material or a carbon material or the like, or a woven fabric, or it may include various additives.

The electrode member 20 includes a base 21 and a plurality of protrusions 22.

The base 21 is a portion of the electrode member 20 for supporting the plurality of protrusions 22. In the example shown in Fig. 1, the base 21 is discoid. Regarding the base 21, in plan view, an outer shape of the base 21 is the same as an outer shape of the support member 10. Here, as shown in Fig. 2, the base 21 includes a first surface 21a and a second surface 21b. The first surface 21a is a surface that is in contact with the plate surface 10a of the support member 10. The first surface 21a is bonded by vulcanization bonding or by an adhesive or the like to the plate surface 10a. The second surface 21b is a surface facing in a direction opposite to a direction in which the first surface 21a faces. The second surface 21b is provided with the plurality of protrusions 22.

It should be noted that, in plan view, a shape of the base 21 is not limited to the example shown in Fig. 1, and it may be, for example, a polygon, or it may differ from the shape of the support member 10 in plan view. Each of the first surface 21a and the second surface 21b is not limited to a flat surface, and it may be a convex curved surface, a concave curved surface, or a conical surface.

Each of the plurality of protrusions 22 is a protrusion protruding from the base 21. A distal end of each of the plurality of protrusions 22 is to be brought into contact with the surface of the living body. Regarding the electrode member 20 including the plurality of protrusions 22, for example, when the bioelectrode 1 is used to measure an electroencephalogram, head hair is pushed aside by the plurality of protrusions 22; accordingly, the distal end of each of the plurality of protrusions 22 is in suitable contact with the scalp. Thus, the biometric signal can be suitably measured.

As shown in Fig. 3, as viewed in a direction of thickness of the base 21, each of the plurality of protrusions 22 is spaced apart from another at regular angular intervals along a virtual circle 25 with a center O of the base 21 as a center. More specifically, a center C1 of a proximal end of each of the plurality of protrusions 22 is disposed on the virtual circle 25. Thus, the plurality of protrusions 22 are arranged in a balanced manner. Here, as viewed in the direction of thickness of the base 21, the plurality of protrusions 22 are disposed to surround a circular region 28 that includes the center O of the base 21. In an example shown in Fig. 3, the number of protrusions 22 is six.

The arrangement of the plurality of protrusions 22 is not limited to the example shown in Fig. 3; for example, it may be an arrangement in which each of the plurality of protrusions 22 is spaced apart from another at irregular angular intervals along the virtual circle 25 as viewed in the direction of thickness of the base 21, or it may be an arrangement in which the center C1 is outside the virtual circle 25, or it may be an arrangement in which each of the plurality of protrusions 22 is arranged on a polygon, such as a virtual triangle or a virtual quadrilateral, with the center O as a center. Moreover, in addition to the plurality of protrusions 22 arranged on the virtual circle 25, at least one protrusion 22 may be provided in the region 28, or multiple protrusions 22 arranged on another virtual circle that is concentric with the virtual circle 25 may be provided. Furthermore, the number of protrusions 22 is not limited to the example shown in Fig. 3, and it may be five or less, or it may be seven or more. However, from a viewpoint of realizing stable contact between the living body and the electrode member 20, the number of the protrusions 22 is preferably three or more, and is more preferably four or more.

Each of the plurality of protrusions 22 extends in a direction slightly inclined relative to the direction of thickness of the base 21 such that a center C2 of the distal end is disposed radially outward apart from the center C1 of the proximal end. This inclination allows an increase in a space between the above-mentioned region 28 and the living body surface, and an advantage is obtained in that it is easy to accommodate the hair in the space, as described below. An angle of this inclination is not particularly limited; in addition, from a viewpoint of ensuring good contact between the living body and the electrode member 20, it is preferably 10 degrees or more, and is more preferably 10 degrees or more and 45 degrees or less, and is still more preferably 10 degrees or more and 30 degrees or less. It should be noted that inclination angles of the plurality of protrusions 22 may be the same as, or be different from, one another. In addition, each of the plurality of protrusions 22 may extend in a direction parallel to the direction of thickness of the base 21.

Each of the plurality of protrusions 22 has a tapered shape. Thus, a width of, or an area of a transverse section of, a protrusion 22 that is any one of the plurality of protrusions 22 is gradually reduced from a proximal end of the protrusion 22 toward a distal end of the protrusion 22. Thus, as described above, in a case in which a direction of protrusion of the protrusion 22 is inclined relative to the direction of thickness of the base 21 so as to increase the space between the region 28 and the living body surface, it is possible to reduce a size of the electrode member 20. It should be noted that the width of the protrusion 22 may be substantially constant from the proximal end toward the distal end.

In the example shown in Fig. 3, a transverse-sectional shape of each of the plurality of protrusions 22 is a circle. In addition, as shown in Fig. 2, a distal surface of each of the plurality of protrusions 22 has a convex curved shape. Thus, it is easy to obtain a good state of contact between the living body and the protrusion 22, or to obtain good usability. It should be noted that a transverse-sectional shape of each of the plurality of protrusions 22 is not limited to a circle, and it may be a polygon such as a square or a pentagon, for example. In addition, a shape of the distal surface of each of the plurality of protrusions 22 is not limited to the shape shown in the drawing, and it may be freely selected.

A height h of the protrusion 22 is not particularly limited, and it is preferably 6 mm or more and 15 mm or less. The height h within such a range allows the distal end of the protrusion 22 to be in suitable contact with the living body surface with the hair. In particular, in a configuration in which any protrusion 22 is not disposed in the region 28 as described above, even in a state in which the subject has a lot of hair, long hair, or thick hair or the like, the height h within such a range allows the hair pushed aside by the protrusion 22 to be accommodated in the space between the region 28 and the living body surface appropriately. As a result, the distal end of the protrusion 22 can be in suitable contact with the living body surface. Here, it is not necessary to excessively press the protrusion 22 against the living body surface; thus, it is also possible to reduce discomfort experienced by the subject. It should be noted that the height h of the protrusion 22 is a length of a portion of the protrusion 22 from the proximal end to the distal end in the direction of thickness of the base 21, in other words, in a normal direction of the second surface 21b.

In contrast to this, if the height h of the protrusion 22 is too short, it is difficult to keep a good state of contact between the distal end of the protrusion 22 and the living body surface with the hair depending on a state of the hair of the subject or the like. On the other hand, if the height h of the protrusion 22 is too long, it is undesirable from a viewpoint of reduction in a size of the bioelectrode 1; moreover, when the electrode member 20 is formed by use of a mold, mold releasability tends to be worse. It should be noted that, from a viewpoint of ensuring a good state of contact between the living body and the electrode member 20, heights h of the plurality of protrusions 22 are preferably equal.

In addition, the electrode member 20 includes a plurality of holes 20a provided corresponding to the plurality of protrusions 22. Each of the plurality of holes 20a is a hole with a bottom that is open to the first surface 21a of the base 21. Each of the plurality of holes 20a passes through the base 21. A hole 20a that is any one of the plurality of holes 20a extends from the first surface 21a to a location of a middle of a corresponding protrusion 22 in a direction of protrusion of the corresponding protrusion 22. The location of the middle of the corresponding protrusion 22 in the direction of protrusion of the corresponding protrusion 22 denotes a location between a proximal end of the corresponding protrusion 22 and a distal end of the corresponding protrusion 22. In the drawings, a width of the hole 20a is substantially constant over the entire hole 20a in a length direction. In addition, a transverse-sectional shape of the hole 20a is a circle. In each of the plurality of holes 20a, a portion of a core 61 having a distal end 61a described below is inserted. It should be noted that a shape of the hole 20a is determined depending on a shape of the core 61 described below and it is not limited to an example shown in the drawings. In addition, the transverse-sectional shape of the hole 20a may differ from a transverse-sectional shape of the core 61.

The connector 30 is a snap-fastener type of male connector. Although not shown, the connector 30 is connected by fitting to a female connector that is electrically connected to the measuring device. As shown in Fig. 1 and Fig. 4, the connector 30 includes a first conductive member 40 and a second conductive member 50. Each of the first conductive member 40 and the second conductive member 50 is a cylinder with a bottom, which has a flange. Each of the first conductive member 40 and the second conductive member 50 is made of a metallic material such as stainless steel, for example. In addition, the first conductive member 40 and the second conductive member 50 are fitted to each other via the through hole 10c of the support member 10.

The first conductive member 40 includes a first cylinder with a bottom 41 and a first flange 42. The first cylinder with a bottom 41 has a shape of a cylinder with a bottom that has an open end 41a, which is one end, and a closed end 41b, which is the other end. An outer circumferential surface of the first cylinder with a bottom 41 has a portion that is closer to the closed end 41b than to the open end 41a, the portion of the outer circumferential surface being provided with a reduced diameter portion 43 that is recessed radially. In addition, an outer diameter of the first cylinder with a bottom 41 is substantially equal to a diameter of the through hole 10c of the support member 10. The first flange 42 has a shape of a flange extending radially outward from the open end 41a. The first flange 42 is slightly inclined relative to a radius direction such that a radially outward end of the first flange 42 approaches closer to the closed end 41b.

In the above-mentioned first conductive member 40, in a state in which the first flange 42 is interposed between the support member 10 and the base 21 of the electrode member 20, the first cylinder with a bottom 41 is inserted in the through hole 10c of the support member 10. Here, a portion of the first cylinder with a bottom 41 protrudes from the plate surface 10b of the support member 10 so as to be capable of being fitted to the second conductive member 50; thus, the closed end 41b and the reduced diameter portion 43 are exposed to the outside of the through hole 10c. In addition, the first flange 42 causes the base 21 to be elastically deformed and is embedded in the base 21.

The second conductive member 50 includes a second cylinder with a bottom 51 and a second flange 52. The second cylinder with a bottom 51 has a shape of a cylinder with a bottom that has an open end 51a, which is one end, and a closed end 51b, which is the other end. The second cylinder with a bottom 51 is shaped to have a diameter that is gradually reduced in a direction from the open end 51a toward the closed end 51b. An inner diameter of the open end 51a is substantially equal to an outer diameter of the reduced diameter portion 43. The second flange 52 has a shape of a flange extending radially outwardly from the open end 51a. The second flange 52 has a portion that is slightly inclined relative to the radius direction such that a radially outward end of the portion of the second flange 52 is farther away from the closed end 51b.

In the above-mentioned second conductive member 50, the second cylinder with a bottom 51 covers the portion of the first cylinder with a bottom 41 protruding from the plate surface 10b of the support member 10 so as to be fit, as described above. Here, the open end 51a is fitted to the reduced diameter portion 43. In this fitted state, the support member 10 is sandwiched between the first flange 42 and the second flange 52; thus, the connector 30 is fixed to the support member 10.

The core group 60 is an aggregate of a plurality of cores 61 for reinforcing the plurality of protrusions 22 of the electrode member 20. Each of the plurality of cores 61 is made of a material having a Young's modulus greater than that of the material of the electrode member 20. Specifically, examples of the material of each of the plurality of cores 61 include a resin material and a metallic material. In a case in which a resin material is used as a material of a core 61 that is any one of the plurality of cores 61, when the core 61 has a complicated shape, it is possible to form the core 61 with high accuracy by injection molding or the like. The resin material is not particularly limited, and it is preferably a material having a melting point higher than molding processing temperatures of the materials of the electrode member 20 from a viewpoint of workability, and more specifically, a super engineering plastic having a melting point of 150 degrees Celsius or more is preferred. In addition, the resin material may include a conductive or insulating inorganic filler. On the other hand, in a case in which a metallic material is used as a material of each of the plurality of cores 61, an advantage is obtained in that bending deformation of the protrusion 22 is allowed, and it is easy to reduce plastic deformation of the protrusion 22. The metallic material is not particularly limited, and it is preferably stainless steel from the viewpoint of corrosion resistance. In addition, the material of each of the plurality of cores 61 may be insulating or conducting, and when the material is conducting, it is possible to contribute to improvement of the sensitivity of the bioelectrode 1. In this case, from the viewpoint of suitably improving the sensitivity of the bioelectrode 1, the core 61 is preferably in contact with either the first conductive member 40 or the second conductive member 50.

The plurality of cores 61 is provided corresponding to the plurality of protrusions 22. At least a portion of each of the plurality of cores 61 is disposed within a corresponding protrusion 22.

Specifically, as shown in Fig. 4, each of the plurality of cores 61 has the shape of a rod that has a distal end 61a and a proximal end 61b as both ends. Each of the plurality of cores 61 is disposed so as to extend in a direction along a direction of protrusion of the corresponding protrusion 22.

A core 61 that is any one of the plurality of cores 61 has a portion that is inserted in a hole 20a of the electrode member 20, the portion of the core 61 having a distal end 61a. Here, the distal end 61a is disposed within a middle of a corresponding protrusion 22 in a direction of protrusion of the corresponding protrusion 22. Thus, no core 61 exists between the distal end 61a and a distal end 22a of the protrusion 22. Consequently, a portion of the protrusion 22, which is disposed ahead of the distal end 61a, is easily deformed by external force compared to a portion of the protrusion 22 within which the core 61 exists. **In** addition, each of the plurality of cores 61 has a portion protruding from the electrode member 20. This portion is disposed in a notch 10d of the support member 10. As described above, in a state in which the core 61 is disposed in the hole 20a and in the notch 10d, a proximal end 61b of the core 61 is in contact with the second flange 52 of the second conductive member 50. Thus, to prevent the core 61 from coming out from the hole 20a, movement of the core 61 in a length direction of the core 61 is restricted by the second conductive member 50. It should be noted that the core 61 may not be in contact with the second conductive member 50. In addition, examples of a method for preventing the core 61 from coming off from the hole 20a include not only a method caused by a configuration in which the core 61 is in contact with the connector 30, but also a method for fixing the core 61 to another member such as the electrode member 20 by an adhesive, and a method for sticking an adhesive tape on the plate surface 10b of the support member 10.

A transverse-sectional shape of the core 61 is a circle. A distal surface of the core 61 is a convex curved surface. Thus, it is possible to reduce stress concentration on the electrode member 20 caused by the core 61. As a result, it is possible to improve durability of the electrode member 20. Here, from a viewpoint of enhancing the effect of reducing the stress concentration, a radius of curvature of the distal surface of the core 61 is preferably equal to half a width W of the core 61.

In an example shown in Fig. 4, the width W of the core 61 is constant over substantially the entire core 61 in the length direction. A specific width W is determined depending on the type of material of the core 61 or the like, and it is not particularly limited; thus, from a viewpoint of ensuring required mechanical strength of the protrusion 22, it is preferably 1/5 or more and 1/2 or less of a width Wa of the protrusion 22 at a location of the distal end 61a of the core 61, or is more preferably 1/4 or more and 1/3 or less. It should be noted that the width W of the core 61 may not be constant; for example, it may gradually decrease toward the distal end 61a. In addition, the transverse-sectional shape of the core 61 is not limited to a circle; for example, it may be an ellipse, a polygon, or the like.

In addition, regarding a core 61 and a protrusion 22 corresponding to each other, a distance d between a distal end 61a of the core 61 and a distal end 22a of the protrusion 22 is preferably 2 mm or more and 3 mm or less. In a state in which the distance d is within such a range, when the distal end 22a of the protrusion 22 is in contact with the surface of a living body, the protrusion 22 is suitably elastically deformed; thus, the subject has a comfortable feeling during use. In addition, in the state in which the distance d is within such a range, it is also possible to ensure required rigidity of the protrusion 22.

The bioelectrode 1 having the above-mentioned configuration is produced as follows, for example. First, the support member 10 is formed by injection molding or the like. On the other hand, each of the first conductive member 40 and the second conductive member 50 included in the connector 30 is formed by press molding or the like. Furthermore, the first cylinder with a bottom 41 of the first conductive member 40 is inserted into the through hole 10c of the support member 10; as a result, an assembly that is constituted of the support member 10 and the first conductive member 40 is obtained.

Thereafter, the electrode member 20 is formed by insert molding in which the assembly is used as an insert. Here, the support member 10 of the assembly is bonded by vulcanization bonding or the like to the electrode member 20, and a recess for accommodating the first flange 42 of the first conductive member 40 is formed on the first surface 21a of the electrode member 20. In addition, after a core 61 is inserted into each of the plurality of holes 20a of the electrode member 20, the second conductive member 50 is mounted to the first conductive member 40. As described above, the bioelectrode 1 is produced.

It should be noted that the production of the electrode member 20 may be performed by insert molding in which the plurality of cores 61, together with, or in place of, the assembly described above, is used as an insert. In this case, each of the plurality of cores 61 is bonded by vulcanization bonding or the like to the electrode member 20. In a case in which the plurality of cores 61, in place of the assembly described above, is used as an insert, the support member 10 and the electrode member 20 should be bonded by an adhesive or the like to each other. In addition, in a case in which the plurality of cores 61 is used as an insert, from a viewpoint of improvement in positioning accuracy of each of the plurality of cores 61, the proximal end 61b preferably has a head portion, a width of which is greater than the width W, in other words, each of the plurality of cores 61 preferably has the shape of a nail.

As described above, the bioelectrode 1 includes the electrode member 20 and the plurality of cores 61. The electrode member 20 is made of the elastic material with conductivity and includes the base 21, which has the shape of a sheet or the shape of a plate, and includes the first surface 21a and the second surface 21b facing in a direction opposite to a direction in which the first surface 21a faces, and the plurality of protrusions 22 protruding from the second surface 21b. The plurality of cores 61 is made of a material having a Young's modulus greater than that of the elastic material and is provided corresponding to two or more protrusions 22 among the plurality of protrusions 22. In addition, the distal end 61a of each of the plurality of cores 61 is disposed within a middle of a corresponding protrusion 22 in a direction of protrusion.

In the above-mentioned bioelectrode 1, a protrusion 22 is provided with a core 61 made of a material having a Young's modulus greater than that of an elastic material of the protrusion 22; thus, it is possible to increase rigidity of the protrusion 22. Accordingly, it is possible to reduce fatigue of the protrusion 22 caused by repeated use. As a result, in a case in which a target portion of a living body has hair, it is possible to suitably push aside the hair by the plurality of protrusions 22 over a long period of time and to cause the distal ends 22a of the plurality of protrusions 22 to be in suitable contact with a surface of a living body. Therefore, it is possible to execute measurement of biometric information over a long period of time.

In addition, since the distal end 61a of each of the plurality of cores 61 is disposed within the middle of the protrusion 22 in the direction of protrusion, it is possible to use flexibility of a distal portion of the protrusion 22. Thus, it is also possible to reduce discomfort experienced by a subject when biometric information is measured.

In this embodiment, as described above, each of the plurality of cores 61 has the shape of a rod extending in the direction of protrusion of the corresponding protrusion 22. Thus, it is possible to realize a configuration in which no core 61 is exposed at a surface of the protrusion 22. As a result, interface between the protrusion 22 and the core 61 does not occur on the surface of the protrusion 22; thus, it is possible to prevent reduction in durability of the protrusion 22 caused by the core 61. In addition, depending on a thickness of the core 61, it is also possible to appropriately cause bending deformation of the protrusion 22 with bending deformation of the core 61. As a result, it is possible to reduce stress concentration that occurs on an area between a portion of the protrusion 22 within which the core 61 exists in the direction of protrusion of the protrusion 22 and a portion of the protrusion 22 within which no core 61 exists in the direction of protrusion of the protrusion 22.

In addition, as described above, each of the plurality of cores 61 is made of metal or resin; thus, it is possible to suitably reinforce a protrusion 22 by a core 61.

Furthermore, as described above, the elastic material of the electrode member 20 includes the rubber material and the conductive particle; thus, it is possible to easily realize the electrode member 20 with a desired conductivity and elasticity.

In addition, as described above, the distance d between the distal end 61a of each of the plurality of cores 61 and the distal end 22a of the corresponding protrusion 22 is preferably within a range of 2.0 mm or more and 3.0 mm or less. In this case, it is possible to obtain a good balance between rigidity of the protrusion 22 and flexibility of a distal portion of the protrusion 22.

Furthermore, as described above, the distal surface of each of the plurality of cores 61 is a convex curved surface. Thus, it is possible to suitably reduce stress concentration on an area between the distal end 61a of the core 61 and the protrusion 22.

In addition, as described above, in a case in which a proximal end of a core 61 that is any one of the plurality of cores 61 is provided with the head portion, a width of which is greater than a width of a middle of the core 61 in a length direction of the core 61, an advantage is obtained in that it is possible to easily improve positioning accuracy of the core 61 when the electrode member is formed by insert molding in which the core 61 is used as an insert.

Furthermore, as described above, the bioelectrode 1 further comprises the support member 10 having a shape of a plate and being in contact with the first surface 21a. The support member 10 has the plurality of notches 10d through which the plurality of cores 61 passes. Thus, after the support member 10 and the electrode member 20 are assembled into the assembly, it is possible to provide the assembly with the cores 61.

In addition, as described above, each of the plurality of protrusions 22 extends in a direction at an angle relative to the direction of thickness of the base 21. In such protrusions 22, in a case in which there is hair on a target portion of a living body, an advantage is obtained in that the hair is easily pushed aside by the plurality of protrusions 22. In addition, since force causing bending deformation is applied to the protrusions 22, fatigue of the protrusions 22 easily occurs when no core 61 is used. Thus, in a configuration in which such a protrusion 22 is provided, an effect caused by provision of the cores 61 is significant.

### 2. Second Embodiment

In the following, a Second Embodiment according to this disclosure will be described. In the embodiment described below, for elements having effects and functions substantially the same as those of the First Embodiment, reference signs used in the descriptions of the First Embodiment are used, and detailed explanations of such elements are omitted as appropriate.

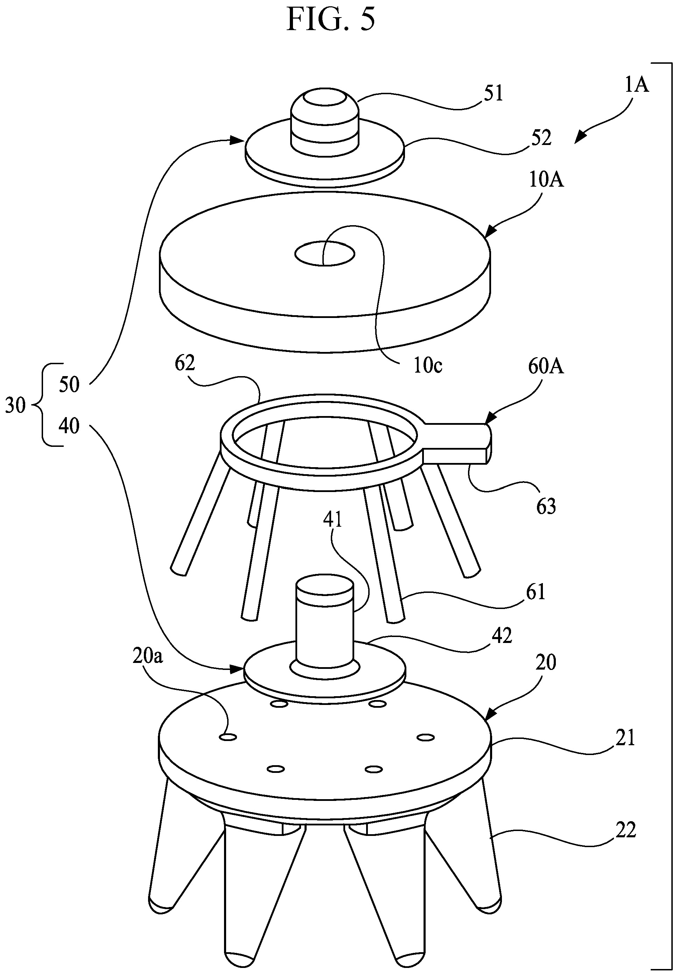

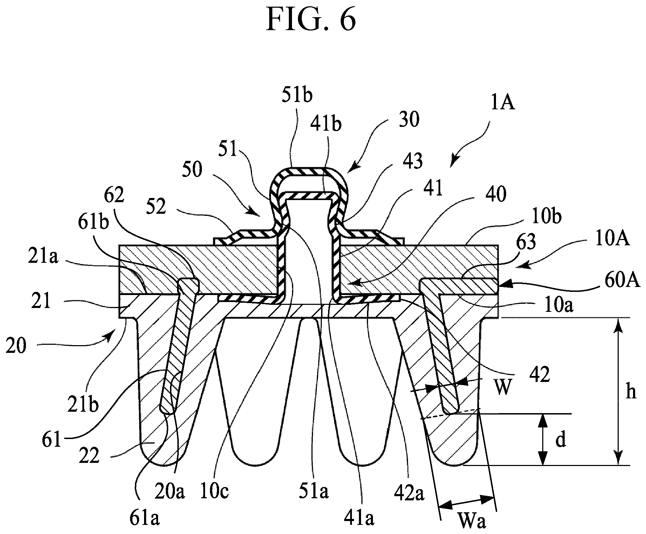

Fig. 5 is an exploded perspective view of a bioelectrode 1A according to the Second Embodiment. Fig. 6 is a cross section of the bioelectrode 1A according to the Second Embodiment. The bioelectrode 1A has the same configuration as the bioelectrode 1 according to the First Embodiment described above, except that a length of each of the plurality of cores 61 differs from that of the First Embodiment and that a support member 10A and a core structure 60A are provided in place of the support member 10 and the core group 60. The support member 10A has the same configuration as the support member 10 according to the First Embodiment, except that the plurality of notches 10d is omitted.

As shown in Fig. 5 and Fig. 6, the core structure 60A includes the plurality of cores 61, an annular member 62, and a protrusion 63. These are integrally made of the same material.

The annular member 62 is a member for supporting the plurality of cores 61. The annular member 62 is interposed between the support member 10A and the electrode member 20. The annular member 62 is connected to the proximal end 61b of each of the plurality of cores 61. The protrusion 63 protrudes radially outward from a portion of the annular member 62 that is in a circumferential direction. The protrusion 63 is used for positioning the core structure 60A when the electrode member 20 is formed by insert molding in which the core structure 60A is used as an insert.

According to the Second Embodiment described above, it is possible to execute measurement of biometric information over a long period of time and to reduce discomfort experienced by a subject when biometric information is measured. In this embodiment, as described above, two or more cores 61 among the plurality of cores 61, are in a single body. The cores 61 having such a configuration have an advantage in that positioning accuracy of the two or more cores 61 is easily enhanced. It should be noted that two or more members obtained by dividing the annular member 62 may be used in place of the annular member 62, and in this case, each of the two or more members allows two or more cores 61 to be in a single body.

In addition, as described above, the bioelectrode 1A further includes the annular member 62 integrated with the plurality of cores 61. Thus, it is possible to increase positioning accuracy of the plurality of cores 61.

Furthermore, as described above, the bioelectrode 1A further includes the support member 10 having a shape of a plate and being in contact with the first surface 21a. In addition, the annular member 62 is interposed between the base 21 and the support member 10. Thus, it is possible to easily form the electrode member 20 by insert molding in which the plurality of cores 61 and the annular member 62 are used as inserts.

### 3. Third Embodiment

In the following, a Third Embodiment according to this disclosure will be described. In the embodiment described below, for elements having effects and functions substantially the same as those of the First Embodiment, reference signs used in the descriptions of the First Embodiment are used, and detailed explanations of such elements are omitted as appropriate.

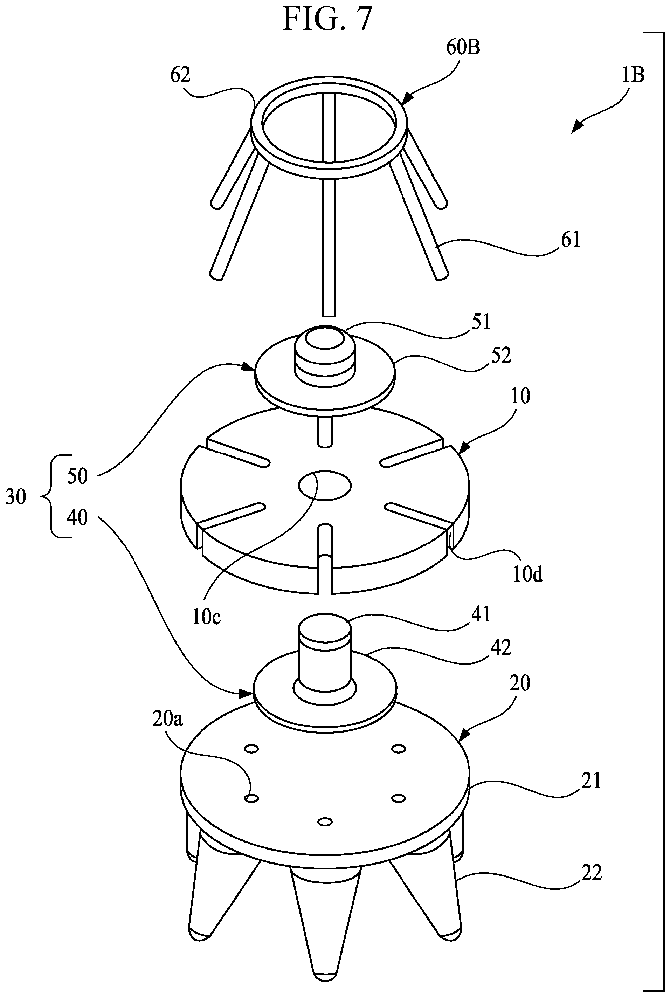

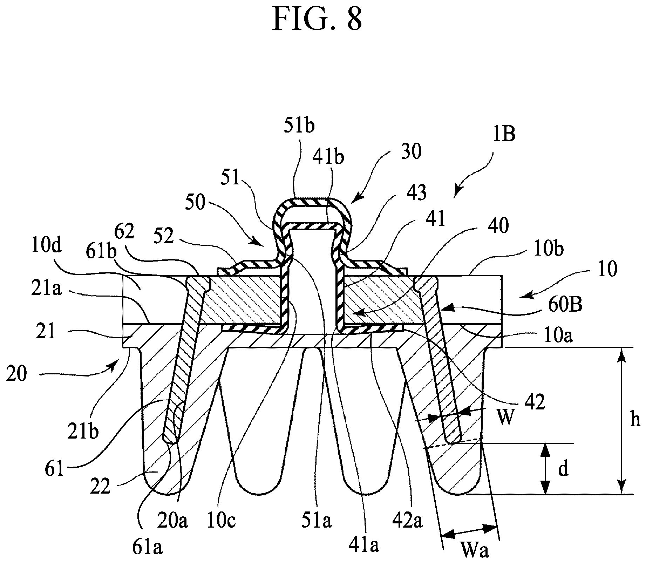

Fig. 7 is an exploded perspective view of a bioelectrode 1B according to the Third Embodiment. Fig. 8 is a cross section of the bioelectrode 1B according to the Third Embodiment. The bioelectrode 1B has the same configuration as the bioelectrode 1 according to the First Embodiment described above, except that a core structure 60B is provided in place of the core group 60.

As shown in Fig. 5 and Fig. 6, the core structure 60B includes the plurality of cores 61 and the annular member 62. These are integrally made of the same material. In other words, the core structure 60B has the same configuration as the core structure 60A according to the Second Embodiment described above, except that a length of each of the plurality of cores 61 differs from that of the Second Embodiment and that the protrusion 63 is omitted.

However, in this embodiment, the support member 10 is interposed between the annular member 62 and the electrode member 20. In the bioelectrode 1B having such a configuration, similar to the First Embodiment, for example, after the support member 10 and the electrode member 20 are assembled into the assembly, the assembly is provided with the core structure 60B.

According to the Third Embodiment described above, it is possible to execute measurement of biometric information over a long period of time and to reduce discomfort experienced by a subject when biometric information is measured. In this embodiment, as described above, the bioelectrode 1B further includes the support member 10 having the shape of a plate, being in contact with the first surface 21a, and being interposed between the base 21 and the annular member 62. In addition, the support member 10 includes plurality of notches 10d through which the plurality of cores 61 passes. Thus, it is possible to assemble the core 61 and the assembly obtained by assembling the support member 10 and the electrode member 20.

### 4. Example of Use

Fig. 9 is a diagram showing an example of use of the bioelectrode 1. In the following, the example of use of the bioelectrode 1 according to the First Embodiment will be described with reference to Fig. 9. It should be noted that the following example of use can be applied to a case in which the bioelectrode 1A or the bioelectrode 1B is used in place of the bioelectrode 1.

As shown in Fig. 9, to measure an electroencephalogram, a plurality of bioelectrodes 1 is appropriately arranged at a plurality of points of the head of a subject H. Here, among the plurality of points, a measurement point Oz is disposed at the back X of the head. In the back X of the head, hairs tend to overlap; thus, it is difficult to obtain a good state of contact between the electrode member 20 and the scalp when the electrode member 20 does not include the plurality of protrusions 22. In contrast to this, in a configuration in which the electrode member 20 includes the plurality of protrusions 22 as described above, it is possible to obtain a good state of contact between the electrode member 20 and the scalp.

### 5. Modifications

Each of the embodiments described above may be variously modified. Specific modifications that may be applied to each of the embodiments described above are described below. Two or more modifications freely selected from the following modifications may be combined as long as no conflict arises from such combination.

### 5-1. First Modification

In each of the embodiments described above, a configuration is described in which each of the plurality of protrusions included in the bioelectrode is provided with a core; however, two or more protrusions among the plurality of protrusions included in the bioelectrode may be provided with cores, and a protrusion that is not provided with any core may exist.

### Description of Reference Signs

1... bioelectrode, 1A... bioelectrode, 1B... bioelectrode, 10... support member, 10A... support member, 10a... plate surface, 10b... plate surface, 10c... through hole, 10d... notch, 20... electrode member, 20a... hole, 21... base, 21a... first surface, 21b... second surface, 22... protrusion, 22a... distal end, 25... virtual circle, 28... region, 30... connector, 40... first conductive member, 41... first cylinder with a bottom, 41a... open end, 41b... closed end, 42... first flange, 43... reduced diameter portion, 50... second conductive member, 51... second cylinder with a bottom, 51a... open end, 51b... closed end, 52... second flange, 60... core group, 60A... core structure, 60B... core structure, 61... core, 61a... distal end, 61b... proximal end, 62... annular member, 63... protrusion, C1... center, C2... center, H... subject, O... center, Oz... measurement point, W... width, Wa... width, X... back of head, d... distance, h... height.

## Claims

1. A bioelectrode comprising:
an electrode member made of elastic material with conductivity, the electrode member including a base having a shape of a sheet or a shape of a plate and including a first surface and a second surface facing in a direction opposite to a direction in which the first surface faces, and a plurality of protrusions protruding from the second surface; and
a plurality of cores made of a material having a Young's modulus greater than that of the elastic material, the plurality of cores being provided corresponding to two or more protrusions among the plurality of protrusions,
wherein a distal end of each of the plurality of cores is disposed within a middle of a corresponding protrusion in a direction of protrusion.

2. The bioelectrode according to Claim 1, wherein each of the plurality of cores has a shape of a rod extending in the direction of protrusion of the corresponding protrusion.

3. The bioelectrode according to Claim 1, wherein each of the plurality of cores is made of metal or resin.

4. The bioelectrode according to Claim 3, wherein the elastic material includes a rubber material and a conductive particle.

5. The bioelectrode according to Claim 1, wherein a distance between the distal end of each of the plurality of cores and a distal end of the corresponding protrusion is within a range of 2.0 mm or more and 3.0 mm or less.

6. The bioelectrode according to Claim 1, wherein a distal surface of each of the plurality of cores is a convex curved surface.

7. The bioelectrode according to Claim 1, wherein a core that is any one of the plurality of cores has a head portion at a proximal end, a width of the head portion being greater than a width of a middle of the core in a direction of length of the core.

8. The bioelectrode according to Claim 1, wherein two or more cores among the plurality of cores are in a single body.

9. The bioelectrode according to Claim 8, further comprising:
an annular member integrated with the plurality of cores.

10. The bioelectrode according to Claim 9, further comprising:
a support member having a shape of a plate and being in contact with the first surface,
wherein the annular member is interposed between the base and the support member.

11. The bioelectrode according to Claim 9, further comprising:
a support member having a shape of a plate, being in contact with the first surface, and being interposed between the base and the annular member,
wherein the support member has a plurality of notches through which the plurality of cores passes.

12. The bioelectrode according to Claim 1, further comprising:
a support member having a shape of a plate and being in contact with the first surface,
wherein the support member has a plurality of notches through which the plurality of cores passes.

13. The bioelectrode according to Claim 1, wherein each of the plurality of protrusions extends in a direction at an angle relative to a direction of thickness of the base.
